# FASCICULE DE BREVET EUROPEEN

(11) **EP 3 648 900 B1**
(45) Date de publication et mention de la délivrance du brevet: **01.09.2021**
(21) Numéro de dépôt: 18749025.5
(22) Date de dépôt: 03.07.2018
(51) Int. Cl.: B05B 11/02, B05B 11/00, B05C 17/005, A61M 15/00, A61M 15/08, A61M 5/32, B65D 83/00, A61M 5/24, A61M 5/30, A61M 11/00

(54) **DISPOSITIF DE DISTRIBUTION DE PRODUIT FLUIDE.**
SPENDER FÜR EIN FLÜSSIGPRODUKT
FLUID PRODUCT DISPENSING DEVICE

(30) Priorité: 06.07.2017 FR 1756400
(43) Date de publication de la demande: 13.05.2020
(73) Titulaire: Aptar France SAS, 27110 Le Neubourg (FR)
(72) Inventeur: ADAM, Fabien, 27930 Aviron (FR)
(74) Mandataire: CAPRI
(86) Numéro de dépôt international: PCT/FR2018/051647
(87) Numéro de publication internationale: WO 2019/008264

(56) Documents cités:
- FR-A1- 2 847 834
- US-A1- 2005 029 288
- US-A1- 2007 000 950
- US-A1- 2008 210 229

## Description

La présente invention concerne un dispositif de distribution de produit fluide, notamment sous forme de spray nasal.

Les dispositifs de distribution de produit fluide sont bien connus dans l'état de la technique.

Généralement, ces dispositifs comportent un réservoir contenant plusieurs doses de fluides à distribuer, sur lequel est monté un organe de distribution, tel qu'une pompe doseuse, pour distribuer une dose à chaque actionnement. Un inconvénient des pompes doseuses concerne l'amorçage, qui impose un ou plusieurs actionnements préalables avant de garantir la distribution d'une dose complète. Ce problème ne se pose pas seulement avant le premier actionnement, mais il peut se poser avant chaque actionnement, surtout s'il se passe du temps, typiquement plusieurs jours, entre deux actionnements successifs. Or, avec certains produits fluides, tels que des produits pharmaceutiques dangereux ou particulièrement onéreux, typiquement pour des traitements anti-migraines, de tels actionnements d'amorçage ne sont pas souhaitables, car ils distribuent nécessairement un peu de fluide, avec donc des risques de mauvais dosage pour l'utilisateur et/ou du gaspillage d'un produit actif qui coûte cher. Par exemple dans un traitement anti-migraine impliquant la distribution successive de quatre doses de médicament, il faut pratiquement remplir le réservoir avec cinq voire six doses de produit fluide pour garantir qu'il y aura bien quatre doses complètes distribuées. Le surcoût en produit actif est donc très important.

Il existe également des dispositifs comportant un réservoir, contenant le produit fluide à distribuer, et un piston, qui est monté coulissant dans ledit réservoir, et qui est déplacé pour distribuer sélectivement le produit fluide contenu dans ledit réservoir. Lorsque le réservoir contient plusieurs doses de produit fluide à distribuer lors de plusieurs actionnements successifs, le déplacement du piston se fait en plusieurs courses d'actionnement successives, de sorte qu'une première dose est distribuée lors d'un premier actionnement, une seconde dose est distribuée lors d'un deuxième actionnement, etc. Avec ce type de dispositif multidose, il se pose le problème de l'altération du produit fluide restant dans le réservoir après la première utilisation. En effet, lors du premier actionnement, le réservoir est ouvert, de sorte que son contenu est susceptible d'être contaminé, notamment lorsque le dispositif est stocké entre deux actionnements successifs. Or, selon le type de produit fluide qui est distribué par le dispositif, notamment lorsqu'il s'agit d'un médicament, il peut être important d'éviter tout risque de contamination.

Les documents FR2847834, US2005029288, US2007000950 et US2008210229 décrivent des dispositifs de l'état de la technique.

La présente invention a pour but de fournir un dispositif de distribution de produit fluide qui ne reproduit pas les inconvénients susmentionnés.

La présente invention a ainsi pour but de fournir un dispositif de distribution de produit fluide qui évite tout gaspillage de produit fluide avant et/ou après chaque actionnement.

La présente invention a aussi pour but de fournir un dispositif de distribution de produit fluide qui assure une protection du produit fluide entre deux actionnements.

La présente invention a également pour but de fournir un tel dispositif de distribution de produit fluide qui soit simple et peu coûteux à fabriquer et à assembler.

La présente invention a donc pour objet un dispositif de distribution de produit fluide comportant un corps pourvu d'un orifice de distribution et d'un élément de perçage, ledit corps contenant un réservoir contenant au moins deux doses de produit fluide, ledit réservoir comportant une ouverture axiale proximale obturée par une membrane et une ouverture axiale distale obturée par un piston, monté coulissant dans ledit réservoir pour distribuer une dose de produit fluide à chaque actionnement, ledit réservoir étant axialement déplaçable par rapport audit corps entre une position de repos, dans laquelle ledit élément de perçage ne traverse pas ladite membrane, et une position de distribution, dans laquelle ledit élément de perçage traverse ladite membrane, un ressort de perçage étant prévu entre ledit corps et ledit réservoir, pour solliciter ledit réservoir vers sa position de repos, ladite membrane étant adaptée à se refermer de manière étanche après chaque actionnement, lorsque ledit élément de perçage ressort dudit réservoir.

Avantageusement, ladite membrane est formée dans une structure comportant au moins deux couches en matières différentes.

Avantageusement, ladite membrane comporte une couche interne, en contact avec le produit fluide, réalisée en butyle.

Avantageusement, ladite couche interne a une épaisseur d'environ 0,6-0,7 mm.

Avantageusement, ladite membrane comporte une couche externe réalisée en polyisoprène.

Avantageusement, ladite couche externe a une épaisseur d'environ 1-1.5 mm.

Avantageusement, ladite membrane est réalisée par calandrage et réticulation, puis découpe, notamment par poinçonnage.

Avantageusement, ledit piston coopère avec une tige de piston qui s'étend axialement hors dudit réservoir, ladite tige de piston coopérant du côté opposé audit piston avec un organe d'actionnement.

Avantageusement, ledit organe d'actionnement est axialement déplaçable dans ledit corps entre une position de repos et une position d'actionnement, l'organe d'actionnement comportant au moins une patte déformable qui coopère avec des dents de ladite tige de piston, pour réaliser les actionnements successifs.

Avantageusement, un ressort de rappel est monté entre l'organe d'actionnement et le corps pour ramener ledit organe d'actionnement dans sa position de repos après chaque actionnement.

Avantageusement, ledit élément de perçage est solidaire d'un insert fixé dans ledit corps, en amont dudit orifice de distribution.

Avantageusement, un profil de pulvérisation est prévu directement en amont dudit orifice de distribution.

Ces avantages et caractéristiques et d'autres de la présente invention apparaitront plus clairement au cours de la description détaillée suivante, faite en référence aux dessins joints, donnés à titre d'exemples non limitatifs, et sur lesquels:
La figure1 est une vue schématique en section transversale d'un dispositif de distribution de produit fluide selon un mode de réalisation avantageux de la présente invention, avant la première utilisation, ,
La figure 2 est une vue similaire à celle de la figure 1, en cours d'actionnement, après perçage du réservoir et avant distribution de la première dose,
La figure 3 est une vue similaire à celle de la figure 2, en cours d'actionnement, pendant la distribution de la première dose,
La figure 4 est une vue similaire à celle de la figure 3, en cours d'actionnement, après la distribution de la première dose et après la fermeture du réservoir, et
La figure 5 est une vue similaire à celle de la figure 4, après actionnement, avec le dispositif prêt pour l'actionnement suivant.

La présente invention sera décrite ci-après en référence à un exemple de réalisation très schématique. Les formes et dimensions des différentes parties constitutives ne sont qu'illustratifs et non limitatifs. Il est entendu que la présente invention s'applique de manière plus générale à tout type de dispositif contenant au moins deux doses.

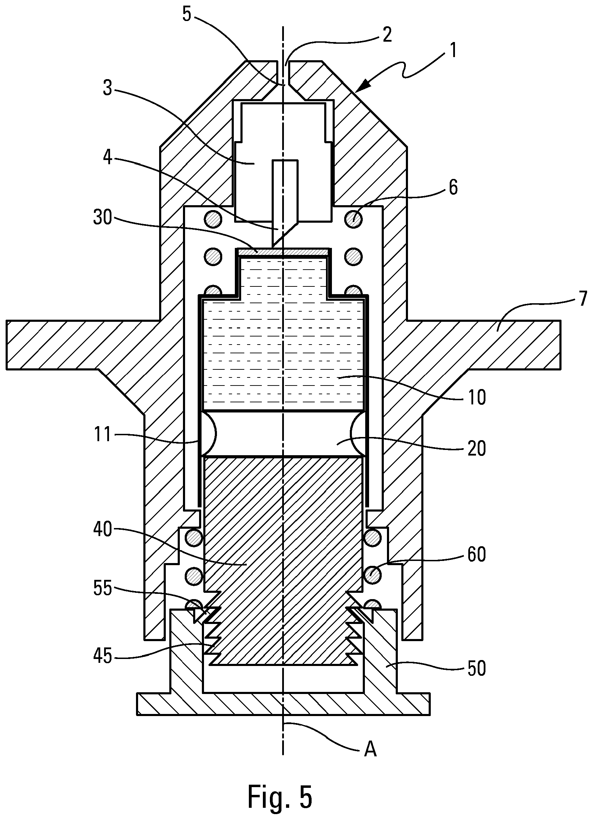

Les termes "proximal" et "distal" se réfèrent à l'orifice de distribution. Le terme "axial" se réfère à l'axe central longitudinal A représenté sur la figure 5.

En se référant aux figures, le dispositif de distribution comporte un corps 1 pourvu d'un orifice de distribution 2.

Ledit corps 1 contient un réservoir 10 contenant plusieurs doses de produit fluide, notamment un médicament liquide, par exemple à pulvériser dans le nez d'un utilisateur. Ce réservoir 10 comporte avantageusement un corps creux 11 sensiblement cylindrique comportant une ouverture axiale proximale et une ouverture axiale distale. L'ouverture distale est obturée de manière étanche par un piston 20, monté coulissant dans ledit réservoir 10. L'ouverture proximale est obturée par une membrane 30. Les dessins montrent une ouverture proximale formée sur une partie cylindrique de diamètre réduit, mais ceci n'est pas obligatoire, et le corps creux 11 formant le réservoir 10 peut aussi être parfaitement cylindrique.

Ledit piston 20 coopère avec une tige de piston 40 qui s'étend axialement hors dudit réservoir 10. Ladite tige de piston 40 coopère donc du côté axial proximal avec ledit piston 20, et elle coopère du côté axial distal avec un organe d'actionnement 50.

Le corps 1 comporte, en amont dudit orifice de distribution 2, un insert 3 monté fixement dans ledit corps 1, ledit insert 3 supportant un élément de perçage creux 4, tel qu'une aiguille ou une canule, destinée à percer ladite membrane 30 du réservoir 10.

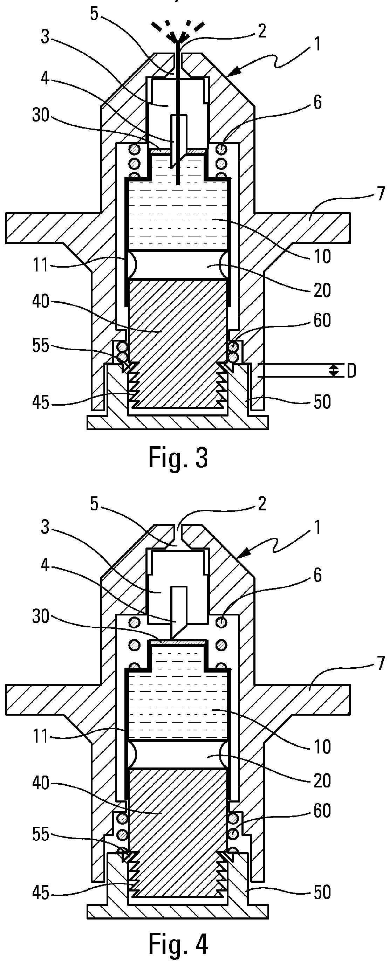

Ledit insert 3 peut également définir un profil de pulvérisation 5 directement en amont dudit orifice de distribution 2, pour générer un spray, notamment un spray nasal. Ce profil de pulvérisation 5 peut être formé de manière classique dans le bord axial proximal dudit insert 3 et/ou dans la paroi de fond dudit corps 1 entourant ledit orifice de distribution 2 et faisant face audit bord axial proximal de l'insert 3. Ce profil de pulvérisation 5 peut comporter des canaux de tourbillonnement et une chambre de tourbillonnement. La figure 3 illustre très schématiquement la distribution d'une dose au moyen d'un trait vertical, mais il est entendu qu'en présence d'un profil de pulvérisation, le fluide passera latéralement entre le corps 1 et l'insert 3, de manière bien connue.

Le corps 1 comporte un élément repose-doigts 7, qui est avantageusement formé de manière monobloc sur ledit corps 1, mais qui en variante pourrait être assemblé autour dudit corps 1.

Le réservoir 10 est monté axialement coulissant dans ledit corps 1 entre une position de repos, dans laquelle ledit élément de perçage 4 ne traverse pas ladite membrane 30, et une position de distribution, dans laquelle ledit élément de perçage 4 traverse ladite membrane 30. Un ressort de perçage 6 est prévu entre ledit corps 1 et ledit réservoir 10, pour solliciter ledit réservoir 10 vers sa position de repos.

Pour réaliser les actionnements successifs de dispositif, ledit organe d'actionnement 50 est axialement déplaçable à l'intérieur dudit corps 1 entre une position de repos et une position d'actionnement. Comme visible sur les figures, l'organe d'actionnement 50 comporte au moins une patte déformable 55 qui est adaptée à coopérer avec des dents 45 de la tige de piston 40, pour réaliser les actionnements successifs.

Un ressort de rappel 60 est monté entre l'organe d'actionnement 50 et le corps 1 pour ramener ledit organe d'actionnement 50 dans sa position de repos après chaque actionnement.

Le fonctionnement du dispositif représenté sur les figures est le suivant.

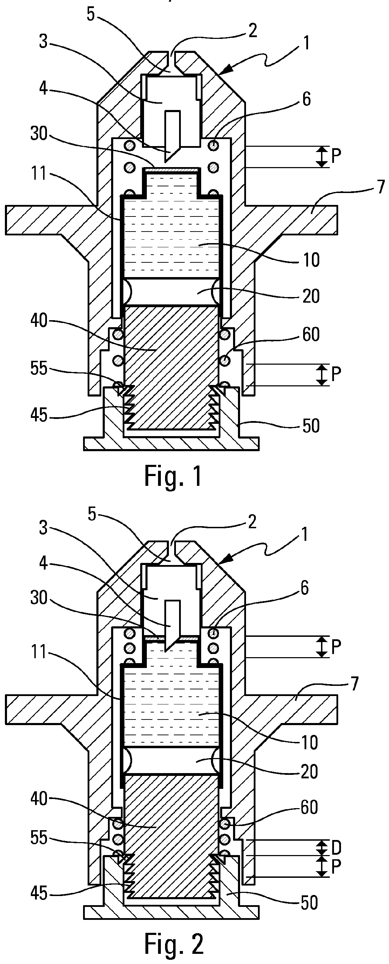

Dans la position de repos de la figure 1, le réservoir 10 est isolé de l'atmosphère d'une part par le piston 20 et d'autre part par la membrane 30. Lorsque l'utilisateur appuie simultanément sur le repose-doigt 7 et sur l'organe d'actionnement 50, il va déplacer ledit organe d'actionnement 50 à l'intérieur du corps 1. La force nécessaire pour déplacer le piston 20 dans le réservoir 10 étant supérieure à celle nécessaire pour comprimer le ressort de perçage 6 et le ressort de rappel 60, le déplacement axial de l'organe d'actionnement 50 va donc déplacer le réservoir 10 et la tige de piston 40 axialement vers l'orifice de distribution 2, comme visible sur la figure 2, avec l'élément de perçage 4 qui va pénétrer dans le réservoir 10 en traversant la membrane 30. Ceci comprime le ressort de perçage 6 et aussi partiellement le ressort de rappel 60. Les figures 1 et 2 illustrent la course de perçage P, identique pour l'organe d'actionnement 50 et pour le réservoir 10. Dans la position de la figure 2, le réservoir 10 coopère avec l'insert 3, qui forme une butée au déplacement axial du réservoir 10. Avantageusement, l'organe d'actionnement 50 coopère pendant cette course de perçage avec le corps 1, par exemple par frottements, pour générer une accumulation d'énergie dans les doigts de l'utilisateur.

Une poursuite du déplacement axial de l'organe d'actionnement 50 en direction de l'orifice de distribution 2 va alors provoquer un déplacement du piston 20 à l'intérieur du réservoir 10 et donc la distribution d'une dose de produit fluide. Le produit fluide est donc poussé par ledit piston 20 à travers l'élément de perçage 4 vers le profil de pulvérisation 5, puis hors du dispositif à travers l'orifice de distribution 2. Avantageusement, l'énergie accumulée dans les doigts de l'utilisateur pendant la course de perçage permet de générer la force suffisante pour déplacer le piston 20 dans le réservoir 10, garantissant ainsi la distribution de la dose complète.

Après distribution d'une dose de produit fluide, le dispositif est dans la position représentée sur la figure 3. L'organe d'actionnement 50 est avantageusement en butée contre une partie du corps 1, ce qui permet de définir avec précision la course de dosage D représentée sur les figures 2 et 3.

Lorsque l'utilisateur relâche la pression sur l'organe d'actionnement 50, le ressort de perçage 6 va d'abord ramener le réservoir 10 vers sa position de repos. L'élément de perçage 4 ressort hors du réservoir 10, et la membrane 30 se referme, pour isoler à nouveau le contenu du réservoir de l'atmosphère. La figure 4 illustre cette position.

Le ressort de rappel 60 va ensuite ramener l'organe d'actionnement 50 vers sa position de repos. Lorsque l'organe d'actionnement 50 revient sous l'effet du ressort de rappel 60 vers sa position de repos, les pattes 55 vont se déformer et venir s'encliqueter sous la prochaine dent 45 de la tige de piston 40, ce qui va permettre à l'utilisateur d'actionner à nouveau le dispositif pour distribuer la prochaine dose de produit fluide.

Selon l'invention, la membrane 30 est adaptée à se refermer de manière étanche après chaque actionnement, lorsque l'élément de perçage 4 ressort du réservoir 10.

Avantageusement, la membrane 30 est formée dans une structure à deux couches, avec deux matières différentes :
- une couche interne, en contact avec le produit fluide, réalisée en butyle, neutre vis-à-vis du produit fluide, typiquement avec une épaisseur d'environ 0,6-0,7 mm,
- une couche externe réalisée en polyisoprène, pour la propriété de fermeture automatique, typiquement avec une épaisseur d'environ 1-1.5 mm.

Avantageusement, la membrane est réalisée par calandrage et réticulation, puis découpe, notamment par poinçonnage. Dans une première variante, les deux matières premières sont calandrées et réticulées ensemble. Dans une seconde variante, une première matière est calandrée et réticulée, puis la seconde matière est calandrée sur la première puis réticulée.

D'autres matériaux ayant des propriétés similaires sont envisageables. De même, une structure monocouche, de préférence en butyle, serait envisageable si le nombre de doses à distribuer est faible, typiquement entre 2 et 5 doses. De même, une membrane avec plus de deux couches serait également possible.

La présente invention fournit donc un dispositif de distribution de produit fluide qui présente notamment les avantages suivants:
- aucun amorçage n'est nécessaire, puisqu'il n'y a pas de chambre de dosage à remplir comme dans une pompe doseuse; le premier actionnement du dispositif donne donc une dose complète;
- pas de perte de produit fluide liée à des actionnements d'amorçage;
- les dimensions du réservoir sont adaptées au nombre de doses à distribuer et à la course de dosage du piston;
- le volume de dose est aisément réglable avec les dimensions du réservoir et/ou la course de dosage;
- le produit fluide contenu dans le réservoir est isolé de l'atmosphère entre deux actionnements, limitant ainsi les risques de contamination bactérienne ou bactériologique.

La présente invention a été décrite en référence à un mode de réalisation avantageux qui n'est pas limitatif, et toute modification utile peut être apportée à la présente invention sans sortir du cadre de celle-ci tel que défini par les revendications annexées.

## Revendications

1. Dispositif de distribution de produit fluide comportant un corps (1) pourvu d'un orifice de distribution (2) et d'un élément de perçage (4), ledit corps (1) contenant un réservoir (10) contenant au moins deux doses de produit fluide, ledit réservoir (10) comportant une ouverture axiale proximale obturée par une membrane (30) et une ouverture axiale distale obturée par un piston (20), monté coulissant dans ledit réservoir (10) pour distribuer une dose de produit fluide à chaque actionnement, **caractérisé en ce que** ledit réservoir (10) est axialement déplaçable par rapport audit corps (1) entre une position de repos, dans laquelle ledit élément de perçage (4) ne traverse pas ladite membrane (30), et une position de distribution, dans laquelle ledit élément de perçage (4) traverse ladite membrane (30), un ressort de perçage (6) étant prévu entre ledit corps (1) et ledit réservoir (10), pour solliciter ledit réservoir (10) vers sa position de repos, ladite membrane (30) étant adaptée à se refermer de manière étanche après chaque actionnement, lorsque ledit élément de perçage (4) ressort dudit réservoir (10).

2. Dispositif selon la revendication 1, dans lequel ladite membrane (30) est formée dans une structure comportant au moins deux couches en matières différentes.

3. Dispositif selon la revendication 2, dans lequel ladite membrane (30) comporte une couche interne, en contact avec le produit fluide, réalisée en butyle.

4. Dispositif selon la revendication 3, dans lequel ladite couche interne a une épaisseur d'environ 0,6-0,7 mm.

5. Dispositif selon l'une quelconque des revendications 2 à 4, dans lequel ladite membrane (30) comporte une couche externe réalisée en polyisoprène.

6. Dispositif selon la revendication 5, dans lequel ladite couche externe a une épaisseur d'environ 1-1.5 mm.

7. Dispositif selon l'une quelconque des revendications 2 à 6, dans lequel ladite membrane (30) est réalisée par calandrage et réticulation, puis découpe, notamment par poinçonnage.

8. Dispositif selon l'une quelconque des revendications précédentes, dans lequel ledit piston (20) coopère avec une tige de piston (40) qui s'étend axialement hors dudit réservoir (10), ladite tige de piston (40) coopérant du côté opposé audit piston (20) avec un organe d'actionnement (50).

9. Dispositif selon la revendication 8, dans lequel ledit organe d'actionnement (50) est axialement déplaçable dans ledit corps (1) entre une position de repos et une position d'actionnement, l'organe d'actionnement (50) comportant au moins une patte déformable (55) qui coopère avec des dents (45) de ladite tige de piston (40), pour réaliser les actionnements successifs.

10. Dispositif selon la revendication 8 ou 9, dans lequel un ressort de rappel (60) est monté entre l'organe d'actionnement (50) et le corps (1) pour ramener ledit organe d'actionnement (50) dans sa position de repos après chaque actionnement.

11. Dispositif selon l'une quelconque des revendications précédentes, dans lequel ledit élément de perçage (4) est solidaire d'un insert (3) fixé dans ledit corps (1), en amont dudit orifice de distribution (2).

12. Dispositif selon l'une quelconque des revendications précédentes, dans lequel un profil de pulvérisation (5) est prévu directement en amont dudit orifice de distribution (2).

## Patentansprüche

1. Vorrichtung zum Verteilen eines Fluidprodukts, die einen Körper (1) aufweist, der mit einer Verteilungsöffnung (2) und einem Durchlochungselement (4) versehen ist, wobei der Körper (1) einen Vorratsbehälter (10) enthält, der wenigstens zwei Dosen des Fluidprodukts enthält, wobei der Vorratsbehälter (10) eine axial proximale Öffnung, die durch eine Membran (30) verschlossen ist, und eine axial distale Öffnung, die durch einen Kolben (20) verschlossen ist, der in dem Vorratsbehälter (10) gleitend montiert ist, um bei jeder Betätigung eine Dosis des Fluidprodukts zu verteilen, aufweist, **dadurch gekennzeichnet, dass** der Vorratsbehälter (10) in Bezug auf den Körper (1) zwischen einer Ruheposition, in der das Durchlochungselement (4) die Membran (30) nicht durchquert, und einer Verteilungsposition, in der das Durchlochungselement (4) die Membran (30) durchquert, axial verlagerbar ist, wobei zwischen dem Körper (1) und dem Vorratsbehälter (10) eine Durchlochungsfeder (6) vorgesehen ist, um den Vorratsbehälter (10) in seine Ruheposition zu drängen, wobei die Membran (30) dafür ausgelegt ist, sich nach jeder Betätigung dicht zu schließen, wenn das Durchlochungselement (4) aus dem Vorratsbehälter (10) austritt.

2. Vorrichtung nach Anspruch 1, wobei die Membran (30) eine Struktur aufweist, die wenigstens zwei Schichten aus unterschiedlichen Materialien enthält.

3. Vorrichtung nach Anspruch 2, wobei die Membran (30) eine innere Schicht aufweist, die mit dem Fluidprodukt in Kontakt ist und aus Butyl hergestellt ist.

4. Vorrichtung nach Anspruch 3, wobei die innere Schicht eine Dicke von etwa 0,6-0,7 mm besitzt.

5. Vorrichtung nach einem der Ansprüche 2 bis 4, wobei die Membran (30) eine äußere Schicht aufweist, die aus Polyisopren hergestellt ist.

6. Vorrichtung nach Anspruch 5, wobei die äußere Schicht eine Dicke von etwa 1-1,5 mm besitzt.

7. Vorrichtung nach einem der Ansprüche 2 bis 6, wobei die Membran (30) durch Kalandrieren und Vernetzen und anschließend durch Ausschneiden, insbesondere durch Lochen, hergestellt wird.

8. Vorrichtung nach einem der vorhergehenden Ansprüche, wobei der Kolben (20) mit einer Kolbenstange (40) zusammenwirkt, die sich axial aus dem Vorratsbehälter (10) erstreckt, wobei die Kolbenstange (40) auf der dem Kolben (20) gegenüberliegenden Seite mit einem Betätigungsorgan (50) zusammenwirkt.

9. Vorrichtung nach Anspruch 8, wobei das Betätigungsorgan (50) in dem Körper (1) zwischen einer Ruheposition und einer Betätigungsposition axial verlagerbar ist, wobei das Betätigungsorgan (50) wenigstens eine verformbare Lasche (55) aufweist, die mit Zähnen (45) der Kolbenstange (40) zusammenwirkt, um die aufeinander folgenden Betätigungen auszuführen.

10. Vorrichtung nach Anspruch 8 oder 9, wobei zwischen dem Betätigungsorgan (50) und dem Körper (1) eine Rückstellfeder (60) montiert ist, um das Betätigungsorgan (50) nach jeder Betätigung in seine Ruheposition zurückzuführen.

11. Vorrichtung nach einem der vorhergehenden Ansprüche, wobei das Durchlochungselement (4) mit einem Einsatz (3), der in dem Körper (1) stromaufseitig der Verteilungsöffnung (2) befestigt ist, fest verbunden ist.

12. Vorrichtung nach einem der vorhergehenden Ansprüche, wobei direkt stromaufseitig der Verteilungsöffnung (2) ein Zerstäubungsprofil (5) vorgesehen ist.

## Claims

1. A fluid dispenser device comprising: a body (1) that is provided with a dispenser orifice (2) and with a perforator element (4), said body (1) containing a reservoir (10) that contains at least two doses of fluid, said reservoir (10) including a proximal axial opening that is closed by a membrane (30) and a distal axial opening that is closed by a piston (20), that is slidably mounted in said reservoir (10) so as to dispense one dose of fluid on each actuation, the dispenser device being **characterized in that** said reservoir (10) is axially movable relative to said body (1) between a rest position in which said perforator element (4) does not pass through said membrane (30), and a dispensing position, in which said perforator element (4) passes through said membrane (30), a perforator spring (6) being provided between said body (1) and said reservoir (10), so as to urge said reservoir (10) towards its rest position, said membrane (30) being adapted to close in leaktight manner after each actuation, when said perforator element (4) withdraws from said reservoir (10).

2. A device according to claim 1, wherein said membrane (30) is formed as a structure comprising at least two layers made out of different materials.

3. A device according to claim 2, wherein said membrane (30) includes an inner layer that is made out of butyl rubber and that is in contact with the fluid.

4. A device according to claim 3, wherein said inner layer has thickness lying in the range about 0.6 mm to about 0.7 mm.

5. A device according to any one of claims 2 to 4, wherein said membrane (30) includes an outer layer that is made out of polyisoprene.

6. A device according to claim 5, wherein said outer layer has thickness lying in the range about 1 mm to about 1.5 mm.

7. A device according to any one of claims 2 to 6, wherein said membrane (30) is made by calendering and cross-linking, and then by cutting out, in particular by punching.

8. A device according to any preceding claim, wherein said piston (20) co-operates with a piston rod (40) that extends axially out from said reservoir (10), said piston rod (40) co-operating, at its end remote from said piston (20), with an actuator member (50).

9. A device according to claim 8, wherein said actuator member (50) is axially movable in said body (1) between a rest position and an actuated position, the actuator member (50) including at least one deformable tab (55) that co-operates with teeth (45) of said piston rod (40), so as to perform successive actuations.

10. A device according to claim 8 or claim 9, wherein a return spring (60) is mounted between the actuator member (50) and the body (1), so as to return said actuator member (50) into its rest position after each actuation.

11. A device according to any preceding claim, wherein said perforator element (4) is secured to an insert (3) that is fastened in said body (1), upstream from said dispenser orifice (2).

12. A device according to any preceding claim, wherein a spray profile (5) is provided directly upstream from said dispenser orifice (2).
